(19) Europäisches Patentamt
European Patent Office
Office européen des brevets

(11) Veröffentlichungsnummer: **0 214 570**
**A2**

(12) # EUROPÄISCHE PATENTANMELDUNG

(21) Anmeldenummer: **86111972.5**

(51) Int. Cl.⁴: **C07K 5/06**, A61K 37/02

(22) Anmeldetag: **29.08.86**

(30) Priorität: **09.09.85 DE 3532035**

(43) Veröffentlichungstag der Anmeldung:
**18.03.87 Patentblatt 87/12**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Urbach, Hansjörg Dr.**
**Le Lavandoustrasse 41**
**D-6242 Kronberg/Taunus(DE)**
Erfinder: **Henning, Rainer, Dr.**
**Rotenhofstrasse 31**
**D-6234 Hattersheim am Main(DE)**
Erfinder: **Becker, Reinhard, Dr.**
**Adelheidstrasse 101**
**D-6200 Wiesbaden(DE)**

(54) **Neue N-alkylierte Tripeptide, Verfahren zu ihrer Herstellung, sie enthaltende Medikamente und deren Verwendung.**

(57) Die Erfindung betrifft N-alkylierte Tripeptide der Formmel I

$$R^3OOC-A-\underset{H}{\underset{|}{N}}-\underset{O}{\underset{\|}{C}}-\underset{R^4}{\underset{|}{CH}}-\underset{R^5}{\underset{|}{N}}-\underset{O}{\underset{\|}{C}}-\underset{R^1}{\underset{|}{CH}}-NH-\underset{COOR^2}{\underset{|}{CH}}-(CH_2)_n-R \qquad (I)$$

worin A den Rest einer Aminosäure bedeutet, $R^4$ zusammen mit $R^5$ und den sie tragenden Atomen ein heterocyclishes Ringsystem bilden, n = 1 oder 2 ist und R, $R^1$, $R^2$ und $R^3$ Wasserstoff oder einen definierten Rest bedeuten, Verfahren zu ihrer Herstellung, sie enthaltende Mittel und deren Verwendung.

EP 0 214 570 A2

# Neue N-alkylierte Tripeptide, Verfahren zu ihrer Herstellung, sie enthaltende Medikamente und deren Verwendung

Die Erfindung betrifft N-alkylierte Tripeptide der Formel I

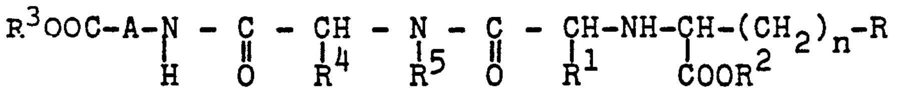

(I)

in welcher

n = 1 oder 2 ist,

R = Wasserstoff,

einen gegebenenfalls substituierten aliphatischen Rest mit 1 bis 8 C-Atomen,

einen gegebenenfalls substituierten alicyclischen Rest mit 3 -9 C-Atomen,

einen gegebenenfalls substituierten aromatischen Rest mit 6 -12 C-Atomen,

einen gegebenenfalls substituierten araliphatischen Rest mit 7 -14 C-Atomen,

einen gegebenenfalls substituierten alicyclischaliphatischen Rest mit 7 - 14 C-Atomen,

einen Rest $OR^a$ oder $SR^a$, worin

$R^a$ für einen gegebenenfalls substituierten aliphatischen Rest mit 1 -4 C-Atomen, für einen gegebenenfalls substituierten aromatischen Rest mit 6 -12 C-Atomen oder einen gegebenenfalls substituierten heteroaromatischen Rest mit 5 -12 Ringatomen steht,

$R^1$ Wasserstoff,

einen gegebenenfalls substituierten aliphatischen Rest mit 1 bis 6 C-Atomen,

einen gegebenenfalls substituierten alicyclischen Rest mit 3 -9 C-Atomen,

einen gegebenenfalls substituierten alicyclischaliphatischen Rest mit 4 -13 C-Atomen,

einen gegebenenfalls substituierten aromatischen Rest mit 6 -12 C-Atomen,

einen gegebenenfalls substituierten araliphatischen Rest mit 7 -16 C-Atomen,

einen gegebenenfalls substituierten heteroaromatischen Rest mit 5 -12 Ringatomen oder

die erforderlichenfalls geschützte Seitenkette einer natürlich vorkommenden α-Aminosäure bedeuten,

$R^2$ und $R^3$ gleich oder verschieden sind und Wasserstoff,

einen gegebenenfalls substituierten aliphatischen Rest mit 1 -6 C-Atomen,

einen gegebenenfalls substituierten alicyclischen Rest mit 3 -9 C-Atomen,

einen gegebenenfalls substituierten aromatischen Rest mit 6 -12 C-Atomen,

einen gegebenenfalls substituierten araliphatischen Rest mit 7 -16 C-Atomen bedeuten und

$R^4$ und $R^5$ zusammen mit den sie tragenden Atomen ein heterocyclisches bi-oder tricyclisches Ringsystem, aus der Gruppe

Octahydrocyclopenta[b]pyrrol (A); 2-Aza-bicyclo-[2,2,2]-octan ( B); 2-Azabicyclo[2,2,1]heptan (C); Spiro[(bicyclo[2,2,1]heptan)-2,3'-pyrrolidin] -(D);Spiro[(bicyclo[2,2,2]-octan)-2,3'-pyrrolidin](E); 2-Azatricyclo[4,3,0,$1^{6,9}$] decan (F); Decahydrocyclohepta[b]pyrrol (G); 2,3,3a,4,5, 7a-Hexahydroindol (H); 2-Azabicyclo[3,1,0]hexan (I); trans-Octahydroindol (J);trans-Octahydroisoindol (K);1,2, 3,3a,4,6a,-Hexahydrocyclopenta[b]pyrrol (L)

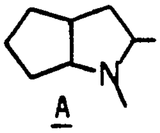
A

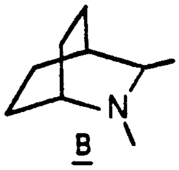
B

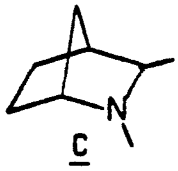
C

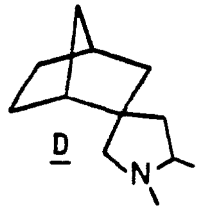
D

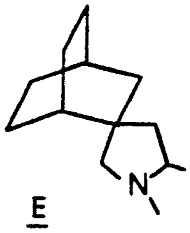
E

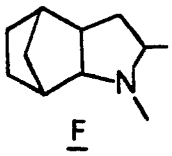
F

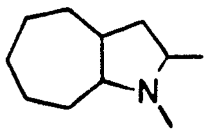
G

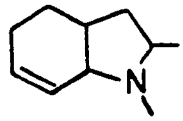
H

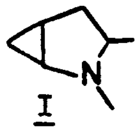
I

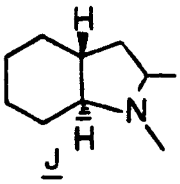
J

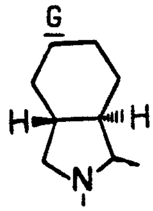
K

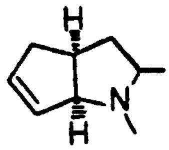
L

und

A den Rest einer gegebenenfalls geschützten natürlichen Aminosäure bedeuten,

sowie deren physiologisch verträglichen Salze mit Säuren und Basen.

Bei den Verbindungen, die mehrere chirale Atome besitzen, kommen alle möglichen Diastereomere als Racemate oder Enantiomere, oder Gemische verschiedener Diastereomere in Betracht.

Bevorzugt werden Verbindungen der Formel I, in welcher

n = 1 oder 2 ist

R Wasserstoff,

Alkyl mit 1 -8 C-Atomen,

Alkenyl mit 2 -6 C-Atomen,

Cycloalkyl mit 3 -9 C-Atomen,

Aryl mit 6 -12 C-Atomen,

das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Acylamino, vorzugsweise ($C_1$-$C_4$)-Alkanoylamino, Methylendioxy, Carboxy, Cyano und/oder Sulfamoyl mono-, di-oder trisubstituiert sein kann,

Alkoxy mit 1 -4 C-Atomen,

Aryloxy mit 6 -12 C-Atomen,

das wie oben bei Aryl beschrieben substituiert sein kann,

mono-bzw. bicyclisches Heteroaryloxy mit 5 -7 bzw. 8 -10 Ringatomen, wovon 1 bis 2 Ringatome Schwefel-oder Sauerstoffatome und/oder 1 bis 4 Ringatome Stickstoff darstellen,

das wie oben bei Aryl beschrieben substituiert sein kann,

Amino-($C_1$-$C_4$)-alkyl,

($C_1$-$C_4$)-Alkanoylamino-($C_1$-$C_4$)alkyl,

($C_7$-$C_{13}$)-Aroylamino-($C_1$-$C_4$)-alkyl,

($C_1$-$C_4$)-Alkoxy-carbonylamino-($C_1$-$C_4$)-alkyl,

($C_6$-$C_{12}$)-Aryl-($C_1$-$C_4$)-alkoxycarbonylamino-($C_1$-$C_4$)-alkyl,

($C_6$-$C_{12}$)-Aryl-($C_1$-$C_4$)-alkylamino-($C_1$-$C_4$)-alkyl,

($C_1$-$C_4$)-Alkylamino-($C_1$-$C_4$)-alkyl,

Di-($C_1$-$C_4$)-alkylamino-($C_1$-$C_4$)-alkyl,

Guanidino-($C_1$-$C_4$)-alkyl,

Imidazolyl, Indolyl,

($C_1$-$C_4$)-Alkylthio,

($C_1$-$C_4$)-Alkylthio-($C_1$-$C_4$)-alkyl,

$(C_6-C_{12})$-Arylthio-$(C_1-C_4$-alkyl,

das im Arylteil wie oben bei Aryl beschrieben, substituiert sein kann,

$(C_6-C_{12})$-Aryl-$(C_1-C_4)$-alkylthio,

das im Arylteil wie oben bei Aryl beschrieben substituiert sein kann,

Carboxy-$(C_1-C_4)$-alkyl,

Carboxy, Carbamoyl,

Carbamoyl-$(C_1-C_4)$-alkyl,

$(C_1-C_4)$-Alkoxy-carbonyl-$(C_1-C_4)$-alkyl,

$(C_6-C_{12})$-Aryloxy-$(C_1-C_4)$-alkyl,

das im Arylteil wie oben bei Aryl beschrieben substituiert sein kann oder

$(C_6-C_{12})$-Aryl-$(C_1-C_4)$-alkoxy,

das im Arylteil wie oben bei Aryl beschrieben substituiert sein kann,

$R^1$ Wasserstoff,

Alkyl mit 1 -6 C-Atomen,

Alkenyl mit 2 -6 C-Atomen,

Alkinyl mit 2 -6 C-Atomen,

Cycloalkyl mit 3 -9 C-Atomen,

Cycloalkenyl mit 5 -9 C-Atomen,

$(C_3-C_9)$-Cycloalkyl-$(C_1-C_4)$-alkyl,

$(C_5-C_9)$-Cycloalkenyl-$(C_1-C_4)$-alkyl,

gegebenenfalls teilhydriertes Aryl mit 6 -12 C-Atomen,

das wie oben bei R beschrieben substituiert sein kann,

$(C_6-C_{12})$-Aryl-$(C_1-C_4)$-alkyl oder $(C_7-C_{13})$-Aroyl-($C_1$ oder $C_2$)alkyl

die beide wie das vorstehende Aryl substituiert sein können

mono-bzw. bicyclisches, gegebenenfalls teilhydriertes Heteroaryl mit 5 -7 bzw. 8 -10 Ringatomen, wovon 1 bis 2 Ringatome Schwefel-oder Sauerstoffatome und/oder 1 bis 4 Ringatome Stickstoffatome darstellen,

das wie das vorstehende Aryl substituiert sein kann oder

die gegebenenfalls geschützte Seitenkette einer natürlich vorkommenden α-Aminosäure R'-CH-$(NH_2)$-COOH bedeuten,

$R^2$ und $R^3$ gleich oder verschieden sind und

Wasserstoff,

Alkyl mit 1 -6 C-Atomen,

Alkenyl mit 2 -6 C-Atomen,

Di-$(C_1-C_4)$-alkylamino-$(C_1-C_4)$-alkyl,

$(C_1-C_5)$-Alkanoyloxy-$(C_1-C_4)$-alkyl,

$(C_1-C_6)$-Alkoxy-carbonyloxy-$(C_1-C_4)$-alkyl,

$(C_7-C_{13})$-Aroyloxy-$(C_1-C_4)$-alkyl,

$(C_6-C_{12})$-Aryloxycarbonyloxy$(C_1-C_4)$-alkyl,

Aryl mit 6 -12 C-Atomen,

$(C_6-C_{12})$-Aryl-$(C_1-C_4)$-alkyl,

$(C_3-C_9)$-Cycloalkyl oder

$(C_3-C_9)$-Cycloalkyl-$(C_1-C_4)$-alkyl

bedeuten und

$R^4$ und $R^5$ die oben angegebene Bedeutung haben

und

A einen Aminosäurerest aus der Gruppe

Alanyl, Leucyl, Isoleucyl, Arginyl, Glycyl, Histidyl, Methionyl, Phenylalanyl, Seryl, Threonyl, Tyrosyl, Valyl, Lysyl, O-Methyltyrosyl, O-Ethyltyrosyl, $N_\epsilon$-tert-Butoxycarbonyl-lysyl, $N_\epsilon$-Benzyloxycarbonyl-lysyl bedeutet.

Besonders bevorzugt sind Verbindungen der Formel I, in welcher

n = 1 oder 2 ist,

R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-

Alkoxycarbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert. Butoxycarbonylamino-($C_1$-$C_4$)-alkyl, Benzoyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono-oder disubstituiert oder im Falle von Methoxy, trisubstituiert sein kann, bedeutet,

$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, $C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono bzw. bicyclischer Heterocyclen-Rest mit 5 bis 7 bzw. 8 bis 10 Ringatomen, wovon 1 bis 2 Ringatome Schwefel-oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, -($C_1$-$C_4$)-Alkyl oder Benzyl bedeuten und

$R^4$ und $R^5$ die oben angegebene Bedeutung haben und

A einen Aminosäurerest aus der Gruppe

Alanyl, Phenylalanyl, Lysyl, N - Benzyloxycarbonylysyl oder Seryl bedeutet.

Insbesondere bevorzugt sind Verbindungen der Formel I, in welcher

n = 2 ist

R Phenyl

$R^1$ Methyl

$R^2$ und $R^3$ Wasserstoff oder gleiche oder verschiedene ($C_1$-$C_6$)-Alkyl-Reste oder ($C_7$-$C_{10}$)-Aralkylreste wie Benzyl oder Nitrobenzyl bedeuten

und

$R^4$, $R^5$ und A die oben angegebene Bedeutung haben.

Unter Aryl ist hier wie im folgenden vorzugsweise gegebenenfalls substituiertes Phenyl, Biphenylyl oder Naphthyl zu verstehen. Entsprechendes gilt für von Aryl abgeleitete Reste wie Aryloxy, Arylthio. Unter Aroyl wird insbesondere Benzoyl verstanden. Aliphatische Reste können geradkettig oder verzweigt sein.

Unter einem mono-bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α-Aminosäuren sind z.B. in Houben-Weyl, Methoden der Organischen Chemie, Bd. XV/1 und XV/2 beschrieben und sind z.B. Ala, Ser, Thr, Val, Leu, Ile, Asp, Asn, Glu, Gln, Cys, Met, Arg, Lys, Hyl, Orn, Cit, Tyr, Phe, Trp und His.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp sind als Schutzgruppen in der Peptidchemie üb lichen Gruppen bevorzugt. Im Falle, daß $R^1$ die geschützte Lysin-Seitenkette bedeutet, werden die bekannten Amino-Schutzgruppen, insbesondere aber Z, Boc oder ($C_1$-$C_6$)-Alkanoyl bevorzugt. Als O-Schutzgruppen für Tyrosin kommen bevorzugt ($C_1$-$C_6$)-Alkyl, insbesondere Methyl oder Ethyl in Frage. Geeignete Schutzgruppen sind beispielsweise beschrieben in Schröder, Lubke, The Peptides, Volume I, New York, London 1965, Seiten 3-270 und in Kontakte Merck 3/79 14-22 und 1/80 23-35.

Von besonderer Bedeutung sind die folgenden Verbindungen:

N-(1-S-Ethoxycarbonyl-3-phenyl-propyl)-S-alanyl-(2S,3aS-6aS)-octahydrocyclopenta[b]pyrrol-2-carbonyl-S-phenylalanin

N-(1-S-Ethoxycarbonyl-3-phenyl-propyl)-S-alanyl-(2S,3aR,6aS)-octahydrocyclopenta[b]pyrrol-2-carbonyl-S-phenylalanin

N-(1-S-Carboxy-3-phenyl-propyl)-S-alanyl-(2S,3aS,6aS)-octahydrocyclopenta[b]pyrrol-2-carbonyl-S-phenylalanin

N-(1-S-Carboxy-3-phenyl-propyl)-S-alanyl-(2S,3aR,6aS)-octahydrocyclopenta[b]pyrrol-2-carbonyl-S-phenylalanin

N-(1-S-Ethoxycarbonyl-3-phenyl-propyl)-S-alanyl-(2S,3aS,6aS)-octahydrocyclopenta[b]pyrrol-2-carbonyl-S-alanin

N-(1-S-Carboxy-3-phenyl-propyl)-S-alanyl-(2S,3a,6aS)-octahydrocyclopenta[b]pyrrol-2-carbonyl-S-alanin

Nα -[N-(1-S-Ethoxycarbonyl-3-phenyl-propyl)-S-alanyl-(2S,3aS,6aS)-octahydrocyclopenta[b]pyrrol-2-carbonyl]-N -benzyloxycarbonyl-S-lysin

Nα -[N-(1-S-Carboxy-3-phenyl-propyl)-S-alanyl-(2S,3aS,6aS)-octahydrocyclopenta[b]pyrrol-2-carbonyl]-S-lysin

N-(1-S-Ethoxycarbonyl-3-phenyl-propyl)-S-alanyl-(2S,3aS,6aS)-octahydrocyclopenta[b]pyrrol-2-carbonyl-S-serin

N-(1-S-Ethoxycarbonyl-3-phenyl-propyl)-S-O-methyl-tyrosyl-(2S,3aS,6aS)-octahydrocyclopenta-[b]pyrrol-2-carbonyl-S-phenylalanin

N-(1-S-Ethoxycarbonyl-3-phenyl-propyl)-S-0-ethyltyrosyl-(2S,3aS,6aS)-octahydrocyclopenta[b]pyrrol-2-carbonyl-S-phenylalanin

N-(1-S-Carboxy-3-phenylpropyl)-S-lysyl-(2S,3aS,6aS)-octahydrocyclopenta[b]pyrrol-2-carbonyl-S-phenylalanin

N-(1-S-Ethoxycarbonyl-3-phenyl-propyl)-S-alanyl-azabicyclo-[2,2,2]octan-2S-carbonyl-S-phenylalanin

N-(1-S-Carboxy-3-phenyl-propyl)-S-alanyl-azabicyclo[2,2,2]-octan-2S-carbonyl-S-phenylalanin

N-(1-S-Ethoxycarbonyl-3-phenyl-propyl)-S-alanyl-azabicyclo-[2,2,1]heptan-2S-exo-carbonyl-S-phenylalanin

N-(1-S-Ethoxycarbonyl-3-phenyl-propyl)-S-alanyl-azabicyclo-[2,2,1]heptan-2S-endo-carbonyl-S-phenylalanin

N-(1-S-Ethoxycarbonyl-3-phenyl-propyl)-alanyl-spiro[bicyclo-[2.2.2]octan-2,3'-pyrrolidin]-5'-S-exo-carbonyl-S-phenylalanin

N-(1-S-Ethoxycarbonyl-3-phenyl-propyl)-S-alanyl-spiro[bicyclo[2.2.2]octan-2,3'-pyrrolidin]-5'-S-endo-carbonyl-S-phenylalanin

N-(1-S-Ethoxycarbonyl-3-phenyl-propyl)-S-alanyl-spiro[bicyclo[2.2.1]heptan-2,3'-pyrrolidin]-5'-S-carbonyl-S-phenylalanin

N-(1-S-Carboxy-3-phenyl-propyl)-S-alanyl-spiro-[bicyclo[2.2.2]-octan-2,3'-pyrrolidin]-5'-S-exo-carbonyl-S-phenylalanin

N-(1-S-Carboxy-3-phenyl-propyl)-S-alanyl-spiro-[bicyclo[2.2.2]-octan-2,3'-pyrrolidin]-5'-S-endo-carbonyl-S-phenylalanin

N-(1-S-Ethoxycarbonyl-3-phenyl-propyl)-S-alanyl-spiro[bicyclo[2.2.2]-octan-2,3'-pyrrolidin]-5'-S-exo-carbonyl-S-alanin

N-(1-S-Ethoxycarbonyl-3-phenyl-propyl)-S-alanyl-spiro[bicyclo[2.2.2]-octan-2,3'-pyrrolidin]-5'-S-endo-carbonyl-S-alanin

N-(1-S-Ethoxycarbonyl-3-phenyl-propyl)-S-alanyl-spiro[bicyclo[2.2.2]-octan-2,3'-pyrrolidin]-5'-S-exo-carbonyl-S-serin

N-(1-S-Ethoxycarbonyl-3-phenyl-propyl)-S-alanyl-spiro[bicyclo[2.2.2]-octan-2,3'-pyrrolidin]-5'-S-endo-carbonyl-S-serin

N-(1-S-Ethoxycarbonyl-3-phenyl-propyl)-S-alanyl-(1R,2S,4S,6S, 7S)-tricyclo[5.2.1.$0^{2,6}$]-3-aza-decan-4-carbonyl-S-phenylalanin

N-(1-S-Carboxy-3-phenyl-propyl)-S-alanyl-(1R,2S,4S,6S,7S)-bicyclo [5.2.1.$0^{2,6}$ ]-3-aza-decan-4-carbonyl-S-phenylalanin

N-(1-S-Ethoxycarbonyl-3-phenyl-propyl)-S-alanyl-(1S,2S,4S,6R, 7R)-bicyclo[5.2.1.$0^{2,6}$]-3-aza-decan-4-carbonyl-S-phenylalanin

N-(1-S-Carboxy-3-phenyl-propyl)-S-alanyl-(1S,2S,4S,6R,7R)-tricyclo[5.2.1.o $^{2,6}$ ]-3-aza-decan-4-carbonyl-S-phenylalanin

N-(1-S-Ethoxycarbonyl-3-phenyl-propyl)-S-alanyl-(1R,2S,4S, 6R,7S)-tricyclo[5.2.1.$0^{2,6}$]-3-aza-decan-4-carbonyl-S-phenylalanin

N-(1-S-Carboxy-3-phenyl-propyl)-S-alanyl-(1R,2S,4S,6R,7S)-tricyclo[5.2.1.0 $^{2,6}$]-3-aza-decan-4-carbonyl-S-phenylalanin

N-(1-S-Ethoxycarbonyl-3-phenyl-propyl)-S-alanyl-1S,2R,4S, 6S,7R)-tricyclo[5.2.1.0$^{2,6}$]-3-aza-decan-4-carbonyl-S-phenylalanin

N-(1-S-Carboxy-3-phenyl-propyl)-S-alanyl-(1S,2R,4S,6S,7R)-tricyclo[5.2.1.0 $^{2,6}$]-3-aza-decan-4-carbonyl-S-phenylalanin

N-(1-S-Ethoxycarbonyl-3-phenyl-propyl)-(S-alanyl-2S,3aS,8aS)-decahydrocyclohepta[b]pyrrol-2-carbonyl-S-phenylalanin

N-(1-S-Carboxy-3-phenyl-propyl)-S-alanyl-(2S,3aS,8aS)-decahydrocyclohepta[b]pyrrol-2-carbonyl-S-phenylalanin

N-(1-S-Ethoxycarbonyl-3-phenyl-propyl)-S-alanyl-2S,3aR,8aR-decahydrocyclohepta[b]pyrrol-2-carbonyl-S-phenylalanin

N-(1-S-Ethoxycarbonyl-3-phenyl-propyl)-S-alanyl-(2S,3aS,7aS)-2,3,3a,4,5,7a-hexahydroindol-2-carbonyl-S-phenylalanin

N-(1-S-Carboxy-3-phenyl-propyl)-S-alanyl-(2S,3aS,7aS)-2,3,3a,4,5,7a-hexahydroindol-2-carbonyl-S-phenylalanin

N-(1-S-Ethoxycarbonyl-3-phenyl-propyl-S-alanyl-(2S,3aR,7aR)-2,3,3a,4,5,7a-hexahydroindol-2-carbonyl-S-phenylalanin

N-(1-S-Ethoxycarbonyl-3-phenyl-propyl)-S-alanyl-(2S,3aS,7aS)-2,3,3a,4,5,7a-hexahydroindol-2-carbonyl-S-alanin

N-(1-S-Ethoxycarbonyl-3-phenyl-propyl)-S-alanyl-(2S,3aS,7aS)-2,3,3a,4,5,7a-hexahydroindol-2-carbonyl-S-serin

N$\alpha$-[N-(1S-Ethoxycarbonyl-3-phenyl-propyl)-S-alanyl-(2S,3aS, 7aS)-2,3,3a,4,5,7a-hexahydroindol-2-carbonyl]-N$_\epsilon$ -benzyloxycarbonyl-S-lysin

N$\alpha$-[N-(1-S-Carboxy-3-phenyl-propyl)-S-alanyl-(2S,3aS,7aS)-2,3,3a,4,5,7a-hexahydroindol-2-carbonyl ]-S-lysin

N-(1-S-Ethoxycarbonyl-3-phenyl-propyl)-S-alanyl-(2S,3aS,7aS)-2,3,3a,4,5,7a-hexahydroindol-2-carbonyl-S-alanin

N-(1-S-Ethoxycarbonyl-3-phenyl-propyl)-S-alanyl-1S,3S,5S-2-azabicyclo[3.1.0]hexan-3-carbonyl-S-phenylalanin

N-(1-S-Ethoxycarbonyl-3-phenyl-propyl)-S-alanyl-(1R,3S,5R-2)-azabicyclo[3.1.0]hexan-3-carbonyl-S-phenylalanin

N-(1-S-Ethoxycarbonyl-3-phenyl-propyl)-S-alanyl-(2S,3aR,7aS)-octahydroindol-2-carbonyl-S-phenylalanin

N-(1-S-Carboxy-3phenyl-propyl)-S-lysyl-(2S,3aR,7aS)-octahydroindol-2-carbonyl-S-phenylalanin

N-(1-S-Carboxy-3-phenyl-propyl)-S-alanyl-(2S,3aR,7aS)-octahydroindol-2-carbonyl-S-phenylalanin

N-(1-S-Ethoxycarbonyl-3-phenyl-propyl)-S-alanyl-(2S,3aR,7aS)-octahydroindol-2-carbonyl-S-alanin

N-(1-S-Ethoxycarbonyl-3-phenyl-propyl)-S-alanyl-(2S,3aR,7aS-octahydroindol-2-carbonyl-S-serin

N$\alpha$-[N-(1-S-Ethoxycarbonyl-3-phenyl-propyl)-S-alanyl-(2S,3aR,7aS)-octahydroindol-2-carbonyl]-N $_\epsilon$-benzyloxycarbonyl-S-lysin

N$\alpha$-[N-(1-S-Carboxy-3-phenyl-propyl)-S-alanyl-(2S,3aR,7aS)-octahydroindol-2-carbonyl]-S-lysin

N-(1-S-Ethoxycarbonyl-3-phenyl-propyl)-S-O-ethyltyrosyl-(2S,3aR,7aS)-octahydroindol-2-carbonyl-S-phenylalanin

N-(1-S-Ethoxycarbonyl-3-phenyl-propyl-S-alanyl-(2S,3aS,7aR)-octahydroindol-2-carbonyl-S-phenylalanin

N-(1-S-Ethoxycarbonyl-3-phenyl-propyl)-S-alanyl-(1S,3aR,7aR)-octahydroisoindol-1-carbonyl-S-phenylalanin

N-(1-S-Carboxy-3-phenyl-propyl)-S-alanyl-(1S,3aR,7aR)-octahydroindol-1-carbonyl-S-phenylalanin

N-(1-S-Ethoxycarbonyl-3-phenyl-propyl)-S-alanyl-(2S,3aS,6aS)-1,2,3,3a,4,6a-hexahydrocyclopenta[b]-pyrrol-2-carbonyl-S-phenylalanin

N-(1-S-Carboxy-3-phenyl-propyl)-S-alanyl-(2S,3aS,6aS)-1,2,3,3a,4,6a-hexahydrocyclopenta[b]-pyrrol-2-carbonyl-S-phenylalanin

N-(1-S-Ethoxycarbonyl-3-phenyl-propyl)-S-alanyl-(2S,3aR,6aR)-1,2,3,3a,4,6a-hexahydrocyclopenta-[b]pyrrol-2-carbonyl-S-phenylalanin

Die Erfindung betrifft ferner ein Verfahren zur Herstellung von Verbindungen der Formel I, dadurch gekennzeichnet, daß man

a) eine Verbindung der Formel II

$$R-(CH_2)_2-\underset{COOR^2}{CH} - NH - \underset{R^1}{CH} - \underset{O}{\overset{}{C}} - \underset{R^5}{N} - \underset{R^4}{CH} - COOH \qquad (II)$$

in der die Reste R, $R^1$, $R^2$, $R^4$, $R^5$ sowie n die gleiche Bedeutung wie in Formel I haben, mit einem Aminosäureester der Formel III

$R^3OOC$ -A -$NH_2$ (III)

in welcher A wie in Formel I definiert ist und $R^3$ für eine veresternde Gruppe, vorzugsweise die Benzyl- oder tert.-Butylgruppe steht, unter an sich bekannten Peptidknüpfungsbedingungen (siehe z.B. Houben-Weyl, Methoden der organischen Chemie, Bd XV, Teil II, S. 1-364, ferner Angew. Chem. 92 129 (1980) sowie US-Patent 4 331 592) umsetzt und gegebenenfalls die Schutzgruppe $R^3$ unter den dafür geeigneten Bedingungen abspaltet sowie gegebenenfalls Reste $R^2$ hydrolytisch oder hydrogenolytisch entfernt, wobei jeweils besonders Hydrogenolyse für Benzylester, Behandlung mit Trifluoressigsäure für tert.-Butylester und alkalische Spaltung für Alkylester in Betracht kommen,

b) eine Verbindung der Formel IV

$$R^3OOC - A - OC - \underset{R^4}{CH} - \underset{R^5}{N} - \underset{O}{C} - \underset{R^1}{CH} - NH_2 \qquad (IV)$$

in welcher $R^1$, $R^3$, $R^4$ und $R^5$ die gleiche Bedeutung wie in Formel I haben, mit einer Verbindung der Formel V

$R^2OOC$ -CH = CH -CO -R (V)

in welcher R und $R^2$ die gleiche Bedeutung wie in Formel I haben, in bekannter Weise in einer Michael-Reaktion (Organikum, 6.Auflage, S. 492, 1967) umsetzt, die Carbonylgruppe z.B. in sauer alkoholischer Lösung mit einem Edelmetallkatalysator, insbesondere Palladium oder Platin auf Aktivkohle bei einem Druck von 20 bis 120 bar hydriert und gegebenenfalls Reste $R^2$ und/oder $R^3$ unter geeigneten Bedingungen (s. oben) abspaltet,

c) eine Verbindung der Formel IV mit einer Verbindung der Formel VI, worin $R^2$ die gleiche Bedeutung wie in Formel I hat und einer Verbindung der Formel VII,

OHC-$COOR^2$ (VI) R-CO-$CH_3$ (VII)

worin R die gleiche Bedeutung wie in Formel I hat, in bekannter Weise in einer Mannich-Reaktion umsetzt (Bull.Soc. Chim. France, 1975, S. 625), und weiter wie unter b) beschrieben verfährt, oder daß man

d) eine Verbindung der Formel IV mit einer Verbindung der Formel VIII worin n, R und $R^2$ die gleiche Bedeutung wie in Formel I haben,

$$R-(CH_2)_n-\underset{O}{C}-COOR^2 \qquad (VIII)$$

beispielsweise gemäß der in J. Amer. Chem. Soc. 93, 2897 (1971), beschriebenen Verfahrensweise umsetzt, die erhaltenen Schiff-Basen reduziert, wobei vorzugsweise Natriumcyanborhydrid verwendet wird, und gegebenenfalls Reste $R^2$ und/oder $R^3$ untergeeigneten Bedingungen (s. oben) abspaltet,

e) eine Verbindung der Formel IV mit einer Verbindung der Formel IX

$$R-(CH_2)_n-\underset{COOR^2}{CH}-OSO_2CF_3 \qquad (IX)$$

in welcher n, R und $R^2$ die gleiche Bedeutung wie in Formel I haben, z.B. analog der in US-Patent 4 525 301 beschriebenen Verfahrensweise umsetzt, und gegebenenfalls Reste $R^2$ und $R^3$ unter geeigneten Bedingungen abspaltet und die so erhaltenen Verbindungen der Formel I gegebenenfalls in ihre physiologisch verträglichen Salze überführt.

Im US-Patent 4,483,850 sind N-Alkyloligopeptide beschrieben, die Hemmstoffe des Angiotensin Converting Enzyms darstellen. Die erfindungsgemäßen Verbindungen unterscheiden sich von den dort beschriebenen in den Resten $R^4$ und $R^5$.

Verbindungen der Formel II sind bekannt - (siehe z.B. EP-A 79 022, EP-A 105 102; EP-A 113 880; EP-A 116 276; EP-A 84 164, EP-A 90 362).

Verbindungen der Formel IV sind Tripeptide, die aus den einzelnen Aminosäurekomponenten nach an sich bekannten Methoden der Peptidchemie (siehe z.B. Houben-Weyl, Methoden der Organischen Chemie, Bd. XV, Teil II, S. 1-364) aufgebaut werden können.

Die erfindungsgemäßen Verbindungen der Formel I liegen, falls $R^3$= Wasserstoff ist, als innere Salze vor. Als amphotere Verbindungen können sie Salze mit Säuren oder Basen bilden. Diese Salze werden in üblicher Weise durch Umsetzen mit einem Äquivalent Säure bzw. Base hergestellt.

Die Verbindungen der Formel I und deren Salze besitzen lang andauernde, intensive blutdrucksenkenden Wirkung. Sie sind starke Hemmer des Angiotensin-Converting-Enzyms (ACE-Hemmer). Sie können zur Bekämpfung des Bluthochdrucks verschiedene Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1-100 mg, vorzugsweise bei 1-40 mg je Einzeldosis bei einem normalgewichtigen erwachsenen Patienten; dies entspricht etwa 15-1300 µg/kg/Tag, vorzugsweise 15-500 µg/kg/Tag. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken-oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glukose-oder Mannitlösungen, oder auch eine Mischung aus den verschiedenen genannten Lösungsmitteln.

Beispiel 1

N-(1-S-Ethoxycarbonyl-3-phenyl-propyl)-S-alanyl-(2S,3aS,6aS)-octahydrocyclopenta[b]pyrrol-2-carbonyl-S-phenylalanin

a) N-(1-S-Ethoxycarbonyl-3-phenyl-propyl)-S-alanyl-(2S,3aS,-6aS)-octahydrocyclopenta[b]pyrrol-2-carbonyl-S-phenylalanin-tert-butylester

1,04 g N-(1-S-Ethoxycarbonyl-3-phenyl-propyl)-S-alanyl-(2S,3aS,6aS)-octahydrocyclopenta[b]-pyrrol-2-carbonsäure und 0,65 g Phenylalanin-tert-butylester-Hydrochlorid werden in 10 ml trockenem Dichlormethan auf -10°C gekühlt, 2,4 ml Triethylamin zugegeben und anschließend 1,6 ml n-Propylphosphonsäureanhydrid (50 %ig in Dichlormethan zugetropft). Nach 1 Stunde bei -10°C wird noch 3 Stunden bei 20°C gerührt, mit Dichlormethan verdünnt und je 1x mit Eiswasser, 5 %iger $KHSO_4$-Lösung und 5 %iger $NaHCO_3$-Lösung gewaschen, mit $MgSO_4$ getrocknet und eingeengt. Das Rohprodukt wird durch Chromatographie an Kieselgel mit Toluol/Ethylacetat (1:1) als Laufmittel gereinigt. Man erhält 0.9 g farbloses Öl.

$[\alpha]_D$ = -20,9° (c = 1, $CH_3OH$)

b) N-(1-S-Ethoxycarbonyl-3-phenyl-propyl)-S-alanyl-(2S,3aS,6aS)-octahydrocyclopenta[b]pyrrol-2-carbonyl-S-phenylalanin

0,8 g des tert.-Butylester aus a) werden mit 5 ml Trifluoressigsäure 90 Minuten gerührt. Nach Einengen werden 20 ml Toluol zugegeben und erneut eingeengt. Der Rückstand wird in 20 ml Methanol/$H_2O$ (4:1) gelöst und mit Amberlite® IRA 93 auf einen pH-Wert von 4,5 gebracht. Nach Absaugen des Ionenaustauschers wird eingeengt und aus Essigester kristallisiert. Man erhält 0, 5 g farblose Kristalle vom Schmp. 142-143°C.

$[\alpha]_D$ = +9,4° (c= 0,5 / $CH_3OH$)

Unter Verwendung geeigneter Ausgangsmaterialien werden entsprechend den in Beispiel 1 angegebenen Vorschriften hergestellt:

N-(1-S-Ethoxycarbonyl-3-phenyl-propyl)-S-alanyl-(2S,3aR,6aS)-octahydrocyclopenta[b]pyrrol-2-carbonyl-S-phenylalanin

N-(1-S-Ethoxycarbonyl-3-phenyl-propyl)-S-alanyl-(2S,3aS,6aS)-octahydrocyclopenta[b]pyrrol-2-carbonyl-S-alanin

Nα-[N-(1-S-Ethoxycarbonyl-3-phenyl-propyl)-S-alanyl-(2S,3aS,6aS)-octahydrocyclopenta[b]pyrrol-2-carbonyl]-N $_\epsilon$-benzyloxycarbonyl-S-lysin

N-(1-S-Ethoxycarbonyl-3-phenyl-propyl)-S-alanyl-(2S,3aS,6aS)-octahydrocyclopenta[b]pyrrol-2-carbonyl-S-serin

N-(1-S-Ethoxycarbonyl-3-phenyl-propyl)-S-O-methyl-tyrosyl-(2S,3aS,6aS)-octahydrocyclopenta-[b]pyrrol-2-carbonyl-S-phenylalanin

N-(1-S-Ethoxycarbonyl-3-phenyl-propyl)-S-O-ethyltyrosyl-2S,3aS,6aS-octahydrocyclopenta[b]pyrrol-2-carbonyl-S-phenylalanin

N-(1-S-Ethoxycarbonyl-3-phenyl-propyl)-S-alanyl-azabicyclo[2.2.2]octan-2S-carbonyl-S-phenylalanin

N-(1-S-Ethoxycarbonyl-3-phenyl-propyl)-S-alanyl-azabicyclo[2.2.1]heptan-2S-exo-carbonyl-S-phenylalanin

N-(1-S-Ethoxycarbonyl-3-phenyl-propyl)-S-alanyl-azabicyclo[2.2.1]heptan-2S-endo-carbonyl-S-phenylalanin

N-(1-S-Ethoxycarbonyl-3-phenyl-propyl)-S-alanyl-spiro[bicyclo [2.2.2]octan-2,3'-pyrrolidin]-5'-S-exo-carbonyl-S-phenylalanin

N-(1-S-Ethoxycarbonyl-3-phenyl-propyl)-S-alanyl-spiro[bicyclo[2.2.2]octan-2,3'-pyrrolidin]-5'-S-endo-carbonyl-S-phenylalanin

N-(1-S-Ethoxycarbonyl-3-phenyl-propyl)-S-alanyl-spiro[bicyclo[2.2.1]heptan-2,3'-pyrrolidin]-5'-S-carbonyl-S-phenylalanin

N-(1-S-Ethoxycarbonyl-3-phenyl-propyl)-S-alanyl-spiro[bicyclo[2.2.2]octan-2,3'-pyrrolidin]-5'-S-exo-carbonyl-S-alanin

N-(1-S-Ethoxycarbonyl-3-phenyl-propyl)-S-alanyl-spiro[bicyclo[2.2.2]octan-2,3'-pyrrolidin]-5'-S-endo-carbonyl-S-alanin

N-(1-S-Ethoxycarbonyl-3-phenyl-propyl)-S-alanyl-spiro[bicyclo[2.2.2]octan-2,3'-pyrrolidin]-5'-S-exo-carbonyl-S-serin

N-(1-S-Ethoxycarbonyl-3-phenyl-propyl)-S-alanyl-spiro[bicyclo[2.2.2]octan-2,3'-pyrrolidin]-5'-S-endo-carbonyl-S-serin

N-(1-S-Ethoxycarbonyl-3-phenyl-propyl)-S-alanyl-(1R,2S,4S-6S,7S)-tricyclo[5.2.1.0 $^{2,6}$]-3-aza-decan-4-carbonyl-S-phenylalanin

N-(1-S-Ethoxycarbonyl-3-phenyl-propyl)-S-alanyl-(1S,2S,4S-6R,7R)-tricyclo[5.2.1.0 $^{2,6}$]-3-aza-decan-4-carbonyl-S-phenylalanin

N-(1-S-Ethoxycarbonyl-3-phenyl-propyl)-S-alanyl-(1R,2S,4S-6R,7S)-tricyclo[5.2.1.0 $^{2,6}$]-3-aza-decan-4-carbonyl-S-phenylalanin

N-(1-S-Ethoxycarbonyl-3-phenyl-propyl)-S-alanyl-(1S,2R,4S-6S,7R)-tricyclo[5.2.1.0 $^{2,6}$]-3-aza-decan-4-carbonyl-S-phenylalanin

N-(1-S-Ethoxycarbonyl-3-phenyl-propyl)-S-alanyl-(2S,3aS,8aS)-decahydrocyclohepta[b]pyrrol-2-carbonyl-S-phenylalanin

N-(1-S-Ethoxycarbonyl-3-phenyl-propyl)-S-alanyl-(2S,3aR,8aR)-decahydrocyclohepta[b]pyrrol-2-carbonyl-S-phenylalanin

N-(1-S-Ethoxycarbonyl-3-phenyl-propyl)-S-alanyl-(2S,3aS,7aS)-2,3,3a,4,5,7a-hexahydroindol-2-carbonyl-S-phenylalanin

N-(1-S-Ethoxycarbonyl-3-phenyl-propyl)-S-alanyl-(2S,3aR,7aR)-2,3,3a,4,5,7a-hexahydroindol-2-carbonyl-S-phenylalanin

N-(1-S-Ethoxycarbonyl-3-phenyl-propyl)-S-alanyl-(2S,3aS,7aS)-2,3,3a,4,5,7a-hexahydroindol-2-carbonyl-S-alanin

N-(1-S-Ethoxycarbonyl-3-phenyl-propyl)-S-alanyl-(2S,3aS,7aS)-2,3,3a,4,5,7a-hexahydroindol-2-carbonyl-S-serin

Nα-[N-(1S-Ethoxycarbonyl-3-phenyl-propyl)-S-alanyl-(2S,3aS, 7aS)-2,3,3a,4,5,7a-hexahydroindol-2-carbonyl]-$N_\epsilon$-benzyloxycarbonyl-S-lysin

N-(1-S-Ethoxycarbonyl-3-phenyl-propyl)-S-alanyl-(2S,3aS,7aS)-2,3,3a,4,5,7a-hexahydroindol-2-carbonyl-S-alanin

N-(1-S-Ethoxycarbonyl-3-phenyl-propyl)-S-alanyl-(1S,3S,5S)-2-azabicyclo[3.1.0]hexan-3-carbonyl-S-phenylalanin

N-(1-S-Ethoxycarbonyl-3-phenyl-propyl)-S-alanyl-(1R,3S,5R)-2-azabicyclo[3.1.0]hexan-3-carbonyl-S-phenylalanin

N-(1-S-Ethoxycarbonyl-3-phenyl-propyl)-S-alanyl-(2S,3aR,7aS)-octahydroindol-2-carbonyl-S-phenylalanin

N-(1-S-Ethoxycarbonyl-3-phenyl-propyl)-S-alanyl-(2S,3aR,7aS)-octahydroindol-2-carbonyl-S-alanin

N-(1-S-Ethoxycarbonyl-3-phenyl-propyl)-S-alanyl-(2S,3aR,7aS)-octahydroindol-2-carbonyl-S-serin

Nα-[N-(1S-Ethoxycarbonyl-3-phenyl-propyl)-S-alanyl-(2S,3aR, 7aS)-octahydroindol-2-carbonyl ]-N $_\epsilon$-benzyloxycarbonyl-S-lysin

N-(1-S-Ethoxycarbonyl-3-phenyl-propyl)-S-O-ethyltyrosyl-(2S,3aR,7aS)-octahydroindol-2-carbonyl-S-phenylalanin

N-(1-S-Ethoxycarbonyl-3-phenyl-propyl)-S-alanyl-(2S,3aS,7aR)-octahydroindol-2-carbonyl-S-phenylalanin

N-(1-S-Ethoxycarbonyl-3-phenyl-propyl)-S-alanyl-(1S,3aR,7aR)-octahydroisoindol-1-carbonyl-S-phenylalanin

N-(1-S-Ethoxycarbonyl-3-phenyl-propyl)-S-alanyl-(2S,3aS,6aS)-1,2,3,3a,4,6a-hexahydrocyclopenta[b]-pyrrol-2-carbonyl-S-phenylalanin

N-(1-S-Ethoxycarbonyl-3-phenyl-propyl)-S-alanyl-(2S,3aR,6aR)-1,2,3,3a,4,6a-hexahydrocyclopenta-[b]pyrrol-2-carbonyl-S-phenylalanin

Beispiel 2

N-(1-S-Carboxy-3-phenyl-propyl)-S-alanyl-(2S,3aS,6aS)-octahydrocyclopenta[b]pyrrol-2-carbonyl-S-phenylalanin

0.23 g N-(1-S-Ethoxycarbonyl-3-phenyl-propyl)-S-alanyl-(2S,3aS,6aS)-octahydrocyclopenta[b]-pyrrol-2-carbonyl-S-phenylalanin werden in 3 ml $H_2O$/Dioxan (1:1) gelöst, mit 1,1 Äquivalenten 1N KOH versetzt und bei Raumtemperatur 5 Stunden gerührt. Man neutralisiert mit 0,1 N HCl und engt ein. Das Rohprodukt wird in $H_2O$ aufgenommen, auf 10 ml Dowex® 50 gegeben und mit 0,5 % Pyridin in Wasser eluiert. Nach Einengen erhält man 0,16 g farbloses amorphes Pulver.

Analog der in Beispiel 2 angegebenen Vorschrift können die nachstehenden Verbindungen unter Verwendung der entsprechenden Ausgangssubstanzen hergestellt werden:

N-(1-S-Carboxy-3-phenyl-propyl)-S-alanyl-(2S,3aR,6aS)-octahydrocyclopenta[b]pyrrol-2-carbonyl-S-phenylalanin

N-(1-S-Carboxy-3-phenyl-propyl)-S-alanyl-(2S,3aS,6aS)-octahydrocyclopenta[b]pyrrol-2-carbonyl-S-alanin

Nα-[N-(1-S-Carboxy-3-phenyl-propyl)-S-alanyl-(2S,3aS,6aS)-octahydrocyclopenta[b]pyrrol-2-carbonyl]-S-lysin

N-(1-S-Carboxy-3-phenylpropyl)-S-lysyl-2S,3aS,6aS-octahydrocyclopenta[b]pyrrol-2-carbonyl-S-phenylalanin

N-(1-S-Carboxy-3-phenyl-propyl)-S-alanyl-azabicyclo[2.2.2]-octan-2S-carbonyl-S-phenylalanin

N-(1-S-Carboxy-3-phenyl-propyl)-S-alanyl-spiro-[bicyclo[2.2.2] octan-2,3'-pyrrolidin]-5'-S-exo-carbonyl-S-phenylalanin

N-(1-S-Carboxy-3-phenyl-propyl)-S-alanyl-spiro-[bicyclo[2.2.2] octan-2,3'-pyrrolidin]-5'-S-endo-carbonyl-S-phenylalanin

N-(1-S-Carboxy-3-phenyl-propyl)-S-alanyl-(1R,2S,4S,6S,7S)-tricyclo[5.2.1.0 $^{2,6}$]-3-aza-decan-4-carbonyl-S-phenylalanin

N-(1-S-Carboxy-3-phenyl-propyl)-S-alanyl-(1S,2S,4S,6R,7R)-tricyclo[5.2.1.0 $^{2,6}$]-3-aza-decan-4-carbonyl-S-phenylalanin

N-(1-S-Carboxy-3-phenyl-propyl)-S-alanyl-(1R,2S,4S,6R,7S)-tricyclo[5.2.1.0 $^{2,6}$]-3-aza-decan-4-carbonyl-S-phenylalanin

N-(1-S-Carboxy-3-phenyl-propyl)-S-alanyl-(1S,2R,4S,6S,7R)-tricyclo[5.2.1.0 $^{2,6}$]-3-aza-decan-4-carbonyl-S-phenylalanin

N-(1-S-Carboxy-3-phenyl-propyl)-S-alanyl-(2S,3aS,8aS)-decahydrocyclohepta[b]pyrrol-2-carbonyl-S-phenylalanin

N-(1-S-Carboxy-3-phenyl-propyl)-S-alanyl-(2S,3aS,7aS)-2,3,3a,4,5,7a-hexahydroindol-2-carbonyl-S-phenylalanin

Nα-[(1-S-Carboxy-3-phenyl-propyl)-S-alanyl-(2S,3aS,7aS)-2,3,3a,4,5,7a-hexahydroindol-2-carbonyl ]-S-lysin

N-(1-S-Carboxy-3-phenyl-propyl)-S-lysyl-(2S,3aR,7aS)-octahydroindol-2-carbonyl-S-phenylalanin

N-(1-S-Carboxy-3-phenyl-propyl)-S-alanyl-(2S,3aR,7aS)-octahydroindol-2-carbonyl-S-phenylalanin

Nα-[N-(1S-Carboxy-3-phenyl-propyl)-S-alanyl-(2S,3aR,7aS)-octahydroindol-2-carbonyl ]-S-lysin

N-(1-S-Carboxy-3-phenyl-propyl)-S-alanyl-(1S,3aR,7aR)-octahydroisoindol-1-carbonyl-S-phenylalanin

N-(1-S-Carboxy-3-phenyl-propyl)-S-alanyl-(2S,3aS,6aS)-1,2,3,3a,4,6a-hexahydrocyclopenta[b]-pyrrol-2-carbonyl-S-phenylalanin

Beispiel 3

N-(1-S-Ethoxycarbonyl-3-phenyl-propyl)-S-alanyl-(2S,3aS,6aS)-octahydrocyclopenta[b]pyrrol-2-carbonyl-S-phenylalanin

a) N-(1-S-Ethoxycarbonyl-3-phenyl-3-oxo-propyl)-S-alanyl-(2S,3aS,6aS)-octahydrocyclopenta[b]pyrrol-2-carbonyl-S-phenylalanin-benzylester

7 mmol S-Alanyl-(2S,3aS,6aS)-octahydrocyclopenta[b]pyrrol-2-carbonyl-S-phenylalanin-benzylester werden zusammen mit 7 mmol 3-Benzoylacrylsäure-ethylester und 8 mmol Triethylamin in Ethanol (50 ml) 24 Stunden bei 20°C gerührt. Nach Einengen werden die gebildeten Diastereomeren durch Chromatographie an Kieselgel mit Essigester/Cyclohexan (1:2) als Laufmittel getrennt.

b) N-(1S-Ethoxycarbonyl-3-phenyl-propyl)-S-alanyl-(2S,3aS, 6aS)-octahydrocyclopenta[b]pyrrol-2-carbonyl-S-phenylalanin

3 mmol der Verbindung aus a) werden in Ethanol (30 ml) gelöst. Nach Zugabe von 1 ml konzentrierter $H_2SO_4$ wird mit Pd/C (10 %) als Katalysator bei 25 atm Wasserstoffdruck 4 Stunden hydriert. Nach Abfiltrieren wird mit Natriumbicarbonat neutralisiert und eingeengt. Das Produkt stimmt in den physikalischen Eigenschaften mit der Verbindung aus Beispiel 1 überein.

Beispiel 4

N-(1S-Ethoxycarbonyl-3-phenyl-propyl)-S-alanyl-(2S,3aS,6aS)-octahydrocyclopenta[b]pyrrol-2-carbonyl-s-phenylalanin

10 mmol Acetophenon, 10 mmol Glyoxylsäureethylester und 10 mmol S-Alanyl-(2S,3aS,6aS)-octahydrocyclopenta[b]pyrrol-2-carbonyl S-phenylalanin-benzylester werden in 30 ml Eisessig 36 Stunden auf 45°C erhitzt. Nach Einengen im Vakuum wird mit Natriumcarbonatlösung neutral gestellt und mit Essigester extrahiert. Die Essigesterphase wird eingeengt und an Kieselgel mit Essigester/Cyclohexan (1:2) als Laufmittel chromatographiert. Das Produkt (entspricht dem Produkt aus Beispiel 3a)) wird, wie in Beispiel 3b) beschrieben, weiter umgesetzt.

Beispiel 5

N-(1S-Ethoxycarbonyl-3-phenyl-propyl)-S-alanyl-(2S,3aS,6aS)-octahydrocyclpenta[b]pyrrol-2-carbonyl-S-phenylalanin

5 mmol S-Alanyl-(2S,3aS,6aS)-octahydrocyclopenta[b]pyrrol-2-carbonyl-S-phenylalanin-tert.-butylester werden in 15 ml wasserfreiem Ethanol gelöst. Man stellt die Lösung mit ethanolischem Kaliumhydroxid auf pH 7,0 und gibt 0.7 g pulverisiertes Molekularsieb (4 Å) und anschließende 5 mmol 2-Keto-4-phenyl-buttersäureethylester dazu. Es wird eine Lösung von 0,6 g Natriumcyanborhydrid in 6 ml wasserfreiem Ethanol langsam zugetropft. Nach einer Reaktionszeit von 20 Stunden bei 20 bis 25°C wird die Lösung filtriert und das Lösungsmittel abdestilliert.

Der Rückstand wird in Essigester/Wasser aufgenommen. Nach den Eindampfen der Essigesterphasen wird der Rückstand an Kieselgel mit Essigester/Cyclohexan (1:2) chromatographiert.

Das gereinigte Produkt wird, wie in Beispiel 1b) beschrieben, mit Trifluoressigsäure umgesetzt.

Die physikalischen Eigenschaften des so erhaltenen Produkts stimmen mit denen der Verbindung aus Beispiel 1b, überein.

Beispiel 6

N-(1S-Ethoxycarbonyl-3-phenyl-propyl)-S-alanyl-(2S,3aS,6aS)-octahydrocyclopenta[b]pyrrol-2-carbonyl-S-phenylalanin

9, 4 mmol S-Alanyl-(2S,3aS,6aS)-octahydrocyclopenta[b]pyrrol-2-carbonyl-S-phenylalanin-tert.-butylester werden zusammen mit 3,4 mmol Triethylamin in 10 ml trockenem Dichlormethan gelöst. Bei 20°C gibt man 1,16 g(3,4 mmol) 4-Phenyl-2-R-trifluormethylsulfonyloxy-buttersäureethylester gelöst in 5 ml trockenem Dichlormethan tropfenweise zu. Nach 1 Stunde bei 20°C wird mit Wasser gewaschen, über Natriumsulfat getrocknet und eingeengt. Das erhaltene Produkt wird, wie in Beispiel 1b) beschrieben, mit Trifluoressigsäure umgesetzt.

Die physikalischen Eigenschaften des so erhaltenen Produkts stimmen mit denen der Verbindung aus Beispiel 1b, überein.

Analog den in den Beispielen 3 bis 6 beschriebenen Verfahren können jeweils die nach Beispiel 1 aufgeführten Verbindungen unter Verwendung geeigneter Ausgangsmaterialien hergestellt werden.

**Ansprüche**

1. Verbindung der Formel I

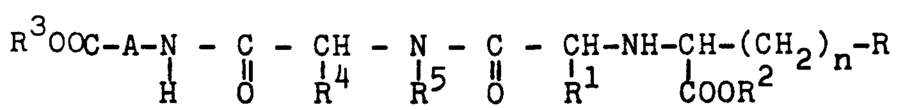

(I)

in welcher

n = 1 oder 2 ist,

R = Wasserstoff,

einen gegebenenfalls substituierten aliphatischen Rest mit 1 bis 8 C-Atomen,

einen gegebenenfalls substituierten alicyclischen Rest mit 3 -9 C-Atomen,

einen gegebenenfalls substituierten aromatischen Rest mit 6 -12 C-Atomen,

einen gegebenenfalls substituierten araliphatischen Rest mit 7 -14 C-Atomen,

einen gegebenenfalls substituierten alicyclischaliphatischen Rest mit 7 -14 C-Atomen,

einen Rest $OR^a$ oder $SR^a$, worin

$R^a$ für einen gegebenenfalls substituierten aliphatischen Rest mit 1 -4 C-Atomen, für einen gegebenenfalls substituierten aromatischen Rest mit 6 -12 C-Atomen oder einen gegebenenfalls substituierten heteroaromatischen Rest mit 5 -12 Ringatomen steht,

$R^1$ Wasserstoff,

einen gegebenenfalls substituierten aliphatischen Rest mit 1 bis 6 C-Atomen,

einen gegebenenfalls substituierten alicyclischen Rest mit 3 -9 C-Atomen,

einen gegebenenfalls substituierten alicyclischaliphatischen Rest mit 4 -13 C-Atomen,

einen gegebenenfalls substituierten aromatischen Rest mit 6 -12 C-Atomen,

einen gegebenenfalls substituierten araliphatischen Rest mit 7 -16 C-Atomen,

einen gegebenenfalls substituierten heteroaromatischen Rest mit 5 -12 Ringatomen oder

die erforderlichenfalls geschützte Seitenkette einer natürlich vorkommenden α-Aminosäure bedeuten,

$R^2$ und $R^3$ gleich oder verschieden sind und Wasserstoff,

einen gegebenenfalls substituierten aliphatischen Rest mit 1 - 6 C-Atomen,

einen gegebenenfalls substituierten alicyclischen Rest mit 3 -9 C-Atomen,

einen gegebenenfalls substituierten aromatischen Rest mit 6 -12 C-Atomen,

einen gegebenenfalls substituierten araliphatischen Rest mit 7 -16 C-Atomen bedeuten und

$R^4$ und $R^5$ zusammen mit den sie tragenden Atomen ein heterocyclisches bi-oder tricyclisches Ringsystem, aus der Gruppe

Octahydrocyclopenta[b]pyrrol (A); 2-Aza-bicyclo-[2.2.2]-octan (B; 2-Azabicyclo[2.2.1]heptan (C); Spiro[(bicyclo[2.2.1]heptan-2,3'-pyrrolidin] (D); Spiro[(bicyclo[2.2.2]-octan)-2,3'-pyrrolidin] (E); 2-Azatricyclo[4.3.0.1$^{6,9}$]-decan (F); Decahydrocyclohepta[b]pyrrol (G); 2,3,3a,4,5, 7a-Hexahydroindol (H); 2-Azabicyclo[3.1.0]hexan (I); trans-Octahydroindol (J); trans-Octahydroisoindol - (K );1,2, 3,3a,4,6a,-Hexahydrocyclopenta[b]pyrrol - (L) und

A den Rest einer gegebenenfalls geschützten natürlichen Aminosäure bedeuten,

sowie deren physiologisch verträglichen Salze mit Säuren oder Basen.

2. Verbindung der Formel I gemäß Anspruch 1, in welcher

n = 1 oder 2 ist

R Wasserstoff,

Alkyl mit 1 -8 C-Atomen,

Alkenyl mit 2 -6 C-Atomen,

Cycloalkyl mit 3 -9 C-Atomen,

Aryl mit 6 -12 C-Atomen,

das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Acylamino, vorzugsweise ($C_1$-$C_4$)-Alkanoylamino, Methylendioxy, Carboxy, Cyano und/oder Sulfamoyl mono-, di-oder trisubstituiert sein kann,

Alkoxy mit 1 -4 C-Atomen,

Aryloxy mit 6 - 12 C-Atomen,

das wie oben bei Aryl beschrieben substituiert sein kann,

mono-bzw. bicyclisches Heteroaryloxy mit 5 -7 bzw. 8 -10 Ringatomen, wovon 1 bis 2 Ringatome Schwefel-oder Sauerstoffatome und/oder 1 bis 4 Ringatome Stickstoff darstellen,

das wie oben bei Aryl beschrieben substituiert sein kann,

Amino-($C_1$-$C_4$)-alkyl,

($C_1$-$C_4$)-Alkanoylamino-($C_1$-$C_4$)alkyl,

($C_7$-$C_{13}$)-Aroylamino-($C_1$-$C_4$)-alkyl,

($C_1$-$C_4$)-Alkoxy-carbonylamino-($C_1$-$C_4$)-alkyl,

($C_6$-$C_{12}$)-Aryl-($C_1$-$C_4$)-alkoxycarbonylamino-($C_1$-$C_4$)-alkyl,

($C_6$-$C_{12}$)-Aryl-($C_1$-$C_4$)-alkylamino-($C_1$-$C_4$)-alkyl,

($C_1$-$C_4$)-Alkylamino-($C_1$-$C_4$)-alkyl,

Di-($C_1$-$C_4$)-alkylamino-($C_1$-$C_4$)-alkyl,

Guanidino-($C_1$-$C_4$)-alkyl,

Imidazolyl, Indolyl,

($C_1$-$C_4$)-Alkylthio,

($C_1$-$C_4$)-Alkylthio-($C_1$-$C_4$)-alkyl,

($C_6$-$C_{12}$)-Arylthio-($C_1$-$C_4$-alkyl,

das im Arylteil wie oben bei Aryl beschrieben, substituiert sein kann,

($C_6$-$C_{12}$)-Aryl-($C_1$-$C_4$)-alkylthio,

das im Arylteil wie oben bei Aryl beschrieben substituiert sein kann,

Carboxy-($C_1$-$C_4$)-alkyl,

Carboxy, Carbamoyl,

Carbamoyl-($C_1$-$C_4$)-alkyl,

($C_1$-$C_4$)-Alkoxy-carbonyl-($C_1$-$C_4$)-alkyl,

($C_6$-$C_{12}$)-Aryloxy-($C_1$-$C_4$)-alkyl,

das im Arylteil wie oben bei Aryl beschrieben substituiert sein kann oder

($C_6$-$C_{12}$)-Aryl-($C_1$-$C_4$)-alkoxy,

das im Arylteil wie oben bei Aryl beschrieben substituiert sein kann,

$R^1$ Wasserstoff,

Alkyl mit 1 -6 C-Atomen,

Alkenyl mit 2 -6 C-Atomen,

Alkinyl mit 2 -6 C-Atomen,

Cycloalkyl mit 3 -9 C-Atomen,

Cycloalkenyl mit 5 -9 C-Atomen,

$(C_3-C_9)$-Cycloalkyl-$(C_1-C_4)$-alkyl,

$(C_5-C_9)$-Cycloalkenyl-$(C_1-C_4)$-alkyl,

gegebenenfalls teilhydriertes Aryl mit 6 -12 C-Atomen,

das wie oben bei R beschrieben substituiert sein kann,

$(C_6-C_{12})$-Aryl-$(C_1-C_4)$-alkyl oder $(C_7-C_{13})$-Aroyl-($C_1$ oder $C_2$)alkyl

die beide wie das vorstehende Aryl substituiert sein können

mono-bzw. bicyclisches, gegebenenfalls teilhydriertes Heteroaryl mit 5 -7 bzw. 8 -10 Ringatomen, wovon 1 bis 2 Ringatome Schwefel-oder Sauerstoffatome und/oder 1 bis 4 Ringatome Stickstoffatome darstellen,

das wie das vorstehende Aryl substituiert sein kann oder

die gegebenenfalls geschützte Seitenkette einer natürlich vorkommenden α-Aminosäure $R^1$-CH-$(NH_2)$-COOH bedeuten,

$R^2$ und $R^3$ gleich oder verschieden sind und

Wasserstoff,

Alkyl mit 1 -6 C-Atomen,

Alkenyl mit 2 -6 C-Atomen,

Di-$(C_1-C_4)$-alkylamino-$(C_1-C_4)$-alkyl,

$(C_1-C_5)$-Alkanoyloxy-$(C_1-C_4)$-alkyl,

$(C_1-C_6)$-Alkoxy-carbonyloxy-$(C_1-C_4)$-alkyl,

$(C_7-C_{13})$-Aroyloxy-$(C_1-C_4)$-alkyl,

$(C_6-C_{12})$-Aryloxycarbonyloxy$(C_1-C_4)$-alkyl,

Aryl mit 6 -12 C-Atomen,

$(C_6-C_{12})$-Aryl-$(C_1-C_4)$-alkyl,

$(C_3-C_9)$-Cycloalkyl oder

$(C_3-C_9)$-Cycloalkyl-$(C_1-C_4)$-alkyl

bedeuten und

$R^4$ und $R^5$ die oben angegebene Bedeutung haben und

A einen Aminosäurerest aus der Gruppe

Alanyl, Leucyl, Isoleucyl, Arginyl, Glycyl, Histidyl, Methionyl, Phenylalanyl, Seryl, Threonyl, Tyrosyl, Valyl, Lysyl, O-Methyltyrosyl, 0-Ethyltyrosyl, $N_\epsilon$tert-Butoxycarbonyl-lysyl, $N_\epsilon$-Benzyloxycarbonyl-lysyl bedeutet

sowie deren physiologisch verträglichen Salze mit Säuren und Basen.

3. Verbindung der Formel I gemäß Anspruch 1 oder 2, in welcher

n= 1 oder 2 ist,

R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di-oder trisubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/oder Methylendioxy mono-oder disubstituiert oder im Falle von Methoxy, trisubstituiert sein kann, bedeutet,

$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, $C_1$ oder $C_2$)-Alkoxy oder Halogen substituiert sein

kann, ($C_6$-$C_{12}$)-Aryl-($C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono-bzw. bicyclischer Heterocyclen-Rest mit 5 bis 7 bzw. 8 bis 10 Ringatomen, wovon 1 bis 2 Ringatome Schwefel-oder Sauerstoffatome und/oder 1 bis 4 Ringatome Stickstoffatome darstellen, oder eine Seitenkette einer natürlich vorkommenden, gegebenenfalls geschützten $\alpha$-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, -($C_1$-$C_4$)-Alkyl oder Benzyl bedeuten und

$R^4$ und $R^5$ die oben angegebene Bedeutung haben und

A einen Aminosäurerest aus der Gruppe

Alanyl, Phenylalanyl, Lysyl, $N_\epsilon$-Benzyloxycarbonyl-lysyl oder Seryl bedeutet.

sowie deren physiologisch verträglichen Salze mit Säuren oder Basen.

4. Verbindung der Formel I gemäß einem der Ansprüche 1 bis 3, in welcher

n = 2 ist

R Phenyl

$R^1$ Methyl

$R^2$ und $R^3$ Wasserstoff oder gleiche oder verschiedene

($C_1$-$C_6$)-Alkyl-Reste oder ($C_7$-$C_{10}$)-Aralkylreste bedeuten

und

$R^4$, $R^5$ und A die oben angegebene Bedeutung haben, sowie deren physiologisch verträglichen Salze mit Säuren oder Basen.

5. Verfahren zur Herstellung einer Verbindung der Formel I gemäß einem der Ansprüche 1 bis 4, dadurch gekennzeichnet, daß man

a) eine Verbindung der Formel II

$$R-(CH_2)_n-\underset{COOR^2}{\underset{|}{CH}} - NH - \underset{R^1}{\underset{|}{CH}} - \underset{O}{\underset{\|}{C}} - \underset{R^5}{\underset{|}{N}} - \underset{R^4}{\underset{|}{CH}} - COOH \qquad (II)$$

in welcher die Reste R, $R^1$, $R^2$, $R^4$ und $R^5$ sowie n die gleiche Bedeutung wie in Formel I haben,

mit einem Aminosäureester der Formel III

$R^3OOC$ -A -$NH_2$ (III)

in welcher A wie in Formel I definiert ist und $R^3$ für eine veresternde Gruppe steht, umsetzt,

gegebenenfalls die Schutzgruppe $R^3$ abspaltet und gegebenenfalls Rest $R^2$ entfernt,

b) eine Verbindung der Formel IV

$$R^3OOC - A - \underset{O}{\underset{\|}{C}} - \underset{R^4}{\underset{|}{CH}} - \underset{R^5}{\underset{|}{N}} - \underset{O}{\underset{\|}{C}} - \underset{R^1}{\underset{|}{CH}} - NH_2 \qquad (IV)$$

in welcher $R^1$, $R^3$, $R^4$ und $R^5$ die gleiche Bedeutung wie in Formel I haben, mit einer Verbindung der Formel V

$$R^2OOC-CH=CH-\underset{\underset{O}{\|}}{C}-R \qquad (V)$$

in welcher R und $R^2$ die gleiche Bedeutung wie in Formel I haben, die Carbonylgruppe hydriert und gegebenenfalls Reste $R^2$ und/oder $R^3$ abspaltet,

c) eine Verbindung der Formel IV mit einer Verbindung der Formel VI und einer Verbindung der Formel VII,

OHC -COOR$^2$ (VI)

$$R-\underset{\underset{O}{\|}}{C}-CH_3 \qquad (VII)$$

worin R und $R^2$ die gleiche Bedeutung wie in Formel I haben, umsetzt und weiter wie unter b) beschrieben verfährt,

d) eine Verbindung der Formel IV mit einer Verbindung der Formel VIII

$$R-(CH_2)_n-\underset{\underset{O}{\|}}{C}-COOR^2 \quad (VIII)$$

worin n, R und $R^2$ die gleiche Bedeutung wie in Formel I haben, umsetzt,

die erhaltene Schiff-Base reduziert und gegebenenfalls Reste $R^2$ und/oder $R^3$ abspaltet, oder

e) eine Verbindung der Formel IV mit einer Verbindung der Formel IX

$$R-(CH_2)_n-\underset{\underset{COOR^2}{|}}{CH}-OSO_2CF_3 \qquad (IX)$$

in welcher n, R und $R^2$ die gleiche Bedeutung wie in Formel I haben, umsetzt und gegebenenfalls Reste $R^2$ und/oder $R^3$ abspaltet,

und die so erhaltene Verbindung der Formel I gegebenenfalls in ihr physiologisch verträgliches Salz überführt.

6. Verbindung gemäß einem der Ansprüche 1 bis 4 zur Anwendung als Heilmittel.

7. Verbindung gemäß einem der Ansprüche 1 bis 4 zur Anwendung als Heilmittel bei der Behandlung des Bluthochdrucks.

8. Pharmazeutisches Mittel enthaltend eine Verbindung gemäß einem der Ansprüche 1 bis 4.

Patentansprüche für den Vertragsstaat AT

1. Verfahren zur Herstellung einer Verbindung der Formel I

$$R^3OOC-A-\underset{\underset{H}{|}}{N}-\underset{\underset{O}{\|}}{C}-\underset{\underset{R^4}{|}}{CH}-\underset{\underset{R^5}{|}}{N}-\underset{\underset{O}{\|}}{C}-\underset{\underset{R^1}{|}}{CH}-NH-\underset{\underset{COOR^2}{|}}{CH}-(CH_2)_n-R \qquad (I)$$

in welcher

n = 1 oder 2 ist,

R = Wasserstoff,

einen gegebenenfalls substituierten aliphatischen Rest mit 1 bis 8 C-Atomen,

einen gegebenenfalls substituierten alicyclischen Rest mit 3 -9 C-Atomen,

einen gegebenenfalls substituierten aromatischen Rest mit 6 -12 C-Atomen,

einen gegebenenfalls substituierten araliphatischen Rest mit 7 -14 C-Atomen,

einen gegebenenfalls substituierten alicyclischaliphatischen Rest mit 7 -14 C-Atomen,

einen Rest $OR^a$ oder $SR^a$, worin

$R^a$ für einen gegebenenfalls substituierten aliphatischen Rest mit 1 -4 C-Atomen, für einen gegebenenfalls substituierten aromatischen Rest mit 6 -12 C-Atomen oder einen gegebenenfalls substituierten heteroaromatischen Rest mit 5 -12 Ringatomen steht,

$R^1$ Wasserstoff,

einen gegebenenfalls substituierten aliphatischen Rest mit 1 bis 6 C-Atomen,

einen gegebenenfalls substituierten alicyclischen Rest mit 3 -9 C-Atomen,

einen gegebenenfalls substituierten alicyclischaliphatischen Rest mit 4 -13 C-Atomen,

einen gegebenenfalls substituierten aromatischen Rest mit 6 -12 C-Atomen,

einen gegebenenfalls substituierten araliphatischen Rest mit 7 -16 C-Atomen,

einen gegebenenfalls substituierten heteroaromatischen Rest mit 5 -12 Ringatomen oder

die erforderlichenfalls geschützte Seitenkette einer natürlich vorkommenden α-Aminosäure bedeuten,

$R^2$ und $R^3$ gleich oder verschieden sind und Wasserstoff,

einen gegebenenfalls substituierten aliphatischen Rest mit 1 -6 C-Atomen,

einen gegebenenfalls substituierten alicyclischen Rest mit 3 -9 C-Atomen,

einen gegebenenfalls substituierten aromatischen Rest mit 6 -12 C-Atomen,

einen gegebenenfalls substituierten araliphatischen Rest mit 7 -16 C-Atomen bedeuten und

$R^4$ und $R^5$ zusammen mit den sie tragenden Atomen ein heterocyclisches bi-oder tricyclisches Ringsystem, aus der Gruppe

Octahydrocyclopenta[b]pyrrol (A); 2-Aza-bicyclo-[2.2.2]-octan ( B); 2-Azabicyclo[2,2,1]heptan (C); Spiro[(bicyclo[2.2.1]heptan)-2,3'-pyrrolidin] -(D);Spiro[(bicyclo[2.2.2]-octan)-2,3'-pyrrolidin](E); 2-Azatricyclo[4.3.0.1$^{6,9}$] decan (F); Decahydrocyclohepta[b]pyrrol (G); 2,3,3a,4,5, 7a-Hexahydroindol (H); 2-Azabicyclo[3.1.0]hexan (I); trans-Octahydroindol (J);trans-Octahydroisoindol (K );1,2, 3,3a,4,6a,-Hexahydrocyclopenta[b]pyrrol (L) und

A den Rest einer gegebenenfalls geschützten natürlichen Aminosäure bedeuten,

sowie deren physiologisch verträglichen Salzen mit Säuren oder Basen, dadurch gekennzeichnet, daß man

a) eine Verbindung der Formel II

$$R-(CH_2)_n-\underset{COOR^2}{\underset{|}{CH}}-NH-\underset{R^1}{\underset{|}{CH}}-\underset{O}{\underset{\|}{C}}-\underset{R^5}{\underset{|}{N}}-\underset{R^4}{\underset{|}{CH}}-COOH \qquad (II)$$

in welcher die Reste R, $R^1$, $R^2$, $R^4$ und $R^5$ sowie n die gleiche Bedeutung wie in Formel I haben,

mit einem Aminosäureester der Formel III

$R^3OOC-A-NH_2$ (III)

in welcher A wie in Formel I definiert ist und $R^3$ für eine veresternde Gruppe steht, umsetzt,

gegebenenfalls die Schutzgruppe $R^3$ abspaltet und gegebenenfalls Rest $R^2$ entfernt,

b) eine Verbindung der Formel IV

$$R^3OOC-A-\underset{O}{\underset{\|}{C}}-\underset{R^4}{\underset{|}{CH}}-\underset{R^5}{\underset{|}{N}}-\underset{O}{\underset{\|}{C}}-\underset{R^1}{\underset{|}{CH}}-NH_2 \qquad (IV)$$

in welcher $R^1$, $R^3$, $R^4$ und $R^5$ die gleiche Bedeutung wie in Formel I haben, mit einer Verbindung der Formel V

$$R^2OOC-CH=CH-\underset{\underset{O}{\|}}{C}-R \quad (V)$$

in welcher R und $R^2$ die gleiche Bedeutung wie in Formel I haben, die Carbonylgruppe hydriert und gegebenenfalls Reste $R^2$ und/oder $R^3$ abspaltet,

c) eine Verbindung der Formel IV mit einer Verbindung der Formel VI und einer Verbindung der Formel VII,

$$OHC-COOR^2 \quad (VI)$$

$$R-\underset{\underset{O}{\|}}{C}-CH_3 \quad (VII)$$

worin R und $R^2$ die gleiche Bedeutung wie in Formel I haben, umsetzt und weiter wie unter b) beschrieben verfährt,

d) eine Verbindung der Formel IV mit einer Verbindung der Formel VIII

$$R-(CH_2)_n-\underset{\underset{O}{\|}}{C}-COOR^2 \quad (VIII)$$

worin n, R und $R^2$ die gleiche Bedeutung wie in Formel I haben, umsetzt,

die erhaltene Schiff-Base reduziert und gegebenenfalls Reste $R^2$ und/oder $R^3$ abspaltet, oder

e) eine Verbindung der Formel IV mit einer Verbindung der Formel IX

$$R-(CH_2)_n-\underset{\underset{COOR^2}{|}}{CH}-OSO_2CF_3 \qquad (IX)$$

in welcher n, R und $R^2$ die gleiche Bedeutung wie in Formel I haben, umsetzt und gegebenenfalls Reste $R^2$ und/oder $R^3$ abspaltet,

und die so erhaltene Verbindung der Formel I gegebenenfalls in ihr physiologisch verträgliches Salz überführt.

2. Verfahren gemäß Anspruch 1, dadurch gekennzeichnet, daß man eine Verbindung der Formel I herstellt, in welcher

n = 1 oder 2 ist

R Wasserstoff,

Alkyl mit 1 -8 C-Atomen,

Alkenyl mit 2 -6 C-Atomen,

Cycloalkyl mit 3 -9 C-Atomen,

Aryl mit 6 -12 C-Atomen,

das durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Acylamino, vorzugsweise $(C_1-C_4)$-Alkanoylamino, Methylendioxy, Carboxy, Cyano und/oder Sulfamoyl mono-, di-oder trisubstituiert sein kann,

Alkoxy mit 1 -4 C-Atomen,

Aryloxy mit 6 -12 C-Atomen,

das wie oben bei Aryl beschrieben substituiert sein kann,

mono-bzw. bicyclisches Heteroaryloxy mit 5 -7 bzw. 8 -10 Ringatomen, wovon 1 bis 2 Ringatome Schwefel-oder Sauerstoffatome und/oder 1 bis 4 Ringatome Stickstoff darstellen,

das wie oben bei Aryl beschrieben substituiert sein kann,

Amino-$(C_1-C_4)$-alkyl,

$(C_1-C_4)$-Alkanoylamino-$(C_1-C_4)$alkyl,

$(C_7-C_{13})$-Aroylamino-$(C_1-C_4)$-alkyl,

$(C_1-C_4)$-Alkoxy-carbonylamino-$(C_1-C_4)$-alkyl,

$(C_6-C_{12})$-Aryl-$(C_1-C_4)$-alkoxycarbonylamino-$(C_1-C_4)$-alkyl,

$(C_6-C_{12})$-Aryl-$(C_1-C_4)$-alkylamino-$(C_1-C_4)$-alkyl,

$(C_1-C_4)$-Alkylamino-$(C_1-C_4)$-alkyl,

Di-$(C_1-C_4)$-alkylamino-$(C_1-C_4)$-alkyl,

Guanidino-$(C_1-C_4)$-alkyl,

Imidazolyl, Indolyl,

$(C_1-C_4)$-Alkylthio,

$(C_1-C_4)$-Alkylthio-$(C_1-C_4)$-alkyl,

$(C_6-C_{12})$-Arylthio-$(C_1-C_4$-alkyl,

das im Arylteil wie oben bei Aryl beschrieben, substituiert sein kann,

$(C_6-C_{12})$-Aryl-$(C_1-C_4)$-alkylthio,

das im Arylteil wie oben bei Aryl beschrieben substituiert sein kann,

Carboxy-$(C_1-C_4)$-alkyl,

Carboxy, Carbamoyl,

Carbamoyl-$(C_1-C_4)$-alkyl,

$(C_1-C_4)$-Alkoxy-carbonyl-$(C_1-C_4)$-alkyl,

$(C_6-C_{12})$-Aryloxy-$(C_1-C_4)$-alkyl,

das im Arylteil wie oben bei Aryl beschrieben substituiert sein kann oder

$(C_6-C_{12})$-Aryl-$(C_1-C_4)$-alkoxy,

das im Arylteil wie oben bei Aryl beschrieben substituiert sein kann,

$R^1$ Wasserstoff,

Alkyl mit 1 -6 C-Atomen,

Alkenyl mit 2 -6 C-Atomen,

Alkinyl mit 2 -6 C-Atomen,

Cycloalkyl mit 3 -9 C-Atomen,

Cycloalkenyl mit 5 -9 C-Atomen,

$(C_3-C_9)$-Cycloalkyl-$(C_1-C_4)$-alkyl,

$(C_5-C_9)$-Cycloalkenyl-$(C_1-C_4)$-alkyl,

gegebenenfalls teilhydriertes Aryl mit 6 -12 C-Atomen, das wie oben bei R beschrieben substituiert sein kann,

$(C_6-C_{12})$-Aryl-$(C_1-C_4)$-alkyl oder $(C_7-C_{13})$-Aroyl-$(C_1$ oder $C_2)$alkyl

die beide wie das vorstehende Aryl substituiert sein können

mono-bzw. bicyclisches, gegebenenfalls teilhydriertes Heteroaryl mit 5 -7 bzw. 8 -10 Ringatomen, wovon 1 bis 2 Ringatome Schwefel-oder Sauerstoffatome und/oder 1 bis 4 Ringatome Stickstoffatome darstellen,

das wie das vorstehende Aryl substituiert sein kann oder

die gegebenenfalls geschützte Seitenkette einer natürlich vorkommenden $\alpha$-Aminosäure $R'$-CH-$(NH_2)$-COOH bedeuten,

$R^2$ und $R^3$ gleich oder verschieden sind und

Wasserstoff

Alkyl mit 1 -6 C-Atomen,

Alkenyl mit 2 -6 C-Atomen,

Di-$(C_1-C_4)$-alkylamino-$(C_1-C_4)$-alkyl,

$(C_1-C_5)$-Alkanoyloxy-$(C_1-C_4)$-alkyl,

$(C_1-C_6)$-Alkoxy-carbonyloxy-$(C_1-C_4)$-alkyl,

$(C_7-C_{13})$-Aroyloxy-$(C_1-C_4)$-alkyl,

$(C_6-C_{12})$-Aryloxycarbonyloxy$(C_1-C_4)$-alkyl,

Aryl mit 6 -12 C-Atomen,

$(C_6-C_{12})$-Aryl-$(C_1-C_4)$-alkyl,

$(C_3-C_9)$-Cycloalkyl oder

$(C_3-C_9)$-Cycloalkyl-$(C_1-C_4)$-alkyl

bedeuten und

$R^4$ und $R^5$ die oben angegebene Bedeutung haben und

A einen Aminosäurerest aus der Gruppe

Alanyl, Leucyl, Isoleucyl, Arginyl, Glycyl, Histidyl, Methionyl, Phenylalanyl, Seryl, Threonyl, Tyrosyl, Valyl, Lysyl, O-Methyltyrosyl, O-Ethyltyrosyl, N-$\epsilon$-tert-Butoxycarbonyl-lysyl, $N_\epsilon$-Benzyloxycarbonyl-lysyl bedeutet,

sowie deren physiologisch verträglichen Salze mit Säuren und Basen.

3. Verfahren gemäß Anspruch 1 oder 2, dadurch gekennzeichnet, daß man eine Verbindung der Formel I herstellt, in welcher

n = 1 oder 2 ist,

R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert. Butoxycarbonylamino-($C_1$-$C_4$)-alkyl, Benzoyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono-oder disubstituiert oder im Falle von Methoxy, trisubstituiert sein kann, bedeutet,

$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, $C_1$ oder $C_2$)-Alkoxy oder Halogen substituiert sein kann, ($C_6$-$C_{12}$)-Aryl-$C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, -($C_1$-$C_4$)-Alkyl oder Benzyl bedeuten und

$R^4$ und $R^5$ die oben angegebene Bedeutung haben und

A einen Aminosäurerest aus der Gruppe

Alanyl, Phenylalanyl, Lysyl, $N_\epsilon$ -Benzyloxycarbonyllysyl oder Seryl bedeutet.

sowie deren physiologisch verträglichen Salze mit Säuren oder Basen.

4. Verfahren gemäß einem der Ansprüche 1 bis 3, dadurch gekennzeichnet, daß man eine Verbindung der Formel I herstellt, in welcher

n = 2 ist,

R Phenyl,

$R^1$ Methyl,

$R^2$ und $R^3$ Wasserstoff oder gleiche oder verschiedene ($C_1$-$C_6$)-Alkyl-Reste oder ($C_7$-$C_{10}$)-Aralkylreste bedeuten

und

$R^4$, $R^5$ und A die oben angegebene Bedeutung haben, sowie deren physiologisch verträglichen Salze mit Säuren oder Basen.

5. Verfahren zur Herstellung eines pharmazeutischen Mittels, enthaltend eine Verbindung der Formel I gemäß einem der Ansprüche 1-4, dadurch gekennzeichnet, daß man diese und einen Träger in eine geeignete Darreichungsform bringt.